Europäisches Patentamt

European Patent Office

Office européen des brevets

(11) Veröffentlichungsnummer: **0 322 742 B1**

(12) **EUROPÄISCHE PATENTSCHRIFT**

(45) Veröffentlichungstag der Patentschrift: **09.03.94**

(21) Anmeldenummer: **88121430.8**

(22) Anmeldetag: **21.12.88**

Die Akte enthält technische Angaben, die nach dem Eingang der Anmeldung eingereicht wurden und die nicht in dieser Patentschrift enthalten sind.

(51) Int. Cl.5: **C08B 15/00**, A61K 39/385, G01N 33/548, B01J 20/32, C08B 17/00

(54) **Verfahren zur Herstellung einer haptenisierten Polysaccharidmatrix.**

(30) Priorität: **24.12.87 DE 3744068**

(43) Veröffentlichungstag der Anmeldung:
**05.07.89 Patentblatt 89/27**

(45) Bekanntmachung des Hinweises auf die Patenterteilung:
**09.03.94 Patentblatt 94/10**

(84) Benannte Vertragsstaaten:
**AT BE CH DE ES FR GB GR IT LI LU NL SE**

(56) Entgegenhaltungen:
**JP-A-62 142 122**
**US-A- 2 609 368**

**PATENT ABSTRACTS OF JAPAN, Band 11, Nr. 369 (C-461)[2816], 2. Dezember 1987, Seite 134 C 461; & JP-A-62 142 122 (GREEN CROSS CORP.) 25-06-1987**

**H. Ambrosius u.a, Immunologie, Gustav Fischer Verlag, Stuttgart 1979, s.167.**

**J.F.Bach, Immunologie, Flammarion Médecine-Sciences, Paris 1976, s.115-116.**

(73) Patentinhaber: **BOEHRINGER MANNHEIM GMBH**

**D-68298 Mannheim(DE)**

(72) Erfinder: **Mang, Thomas, Dr. rer. nat.**
**Saalangerstrasse 40**
**D-8122 Penzberg(DE)**

(74) Vertreter: **Huber, Bernhard, Dipl.-Chem. et al Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08 20**
**D-81635 München (DE)**

**Beschreibung**

Die Erfindung betrifft ein Verfahren zur Herstellung einer Polysaccharid-haltigen Matrix, an die Haptene kovalent gebunden sind, sowie eine hierzu geeignete Vorrichtung.

Trägermaterialien, an die Haptene adsorptiv oder kovalent gebunden sind, werden sowohl bei der Durchführung immunologischer Nachweisverfahren nach dem Prinzip des Immunoassays als auch für die Affinitäts-Chromatographie verwendet. Dazu ist es erforderlich, daß die Bindung der Haptene an die Matrix so stark ist, daß sie unter den in der Affinitäts-Chromatographie bzw. bei der Durchführung des Immunoassays angewandten Bedingungen nicht abgelöst werden.

Bei der Affinitätschromatographie werden Haptene an einen Träger gebunden, um mit diesem Hapten spezifisch bindefähige Substanzen, wie z. B. gegen das Hapten gerichtete Antikörper, aus einem Gemisch abzutrennen. Nach der Auftrennung des Gemisches z.B. in einer Chromatographiesäule muß die gebundene spezifisch bindefähige Substanz wieder vom Hapten eluiert werden. Dabei darf aber das Hapten nicht abgelöst werden.

Bei Immunoassays dient das an eine feste Phase gebundene Hapten dazu, einen zu bestimmenden Komplex aus einer Reaktionslösung abzutrennen durch Bindung mit einer mit dem Hapten spezifisch bindefähigen Substanz, wie z.B. einem gegen das Hapten gerichteten Antikörper. Auch hier ist es wesentlich, daß das Hapten an dem Träger gebunden bleibt, da ansonsten die Ergebnisse verfälscht werden.

Aus JP-A-62-142 122 ist ein Verfahren zur Bindung von Hepatitis-Virus-B-Oberflächenantigen an Polysaccharid zur Herstellung eines Hepatitis-B-Vakzins bekannt, bei welchem ein Polysaccharid mit einem Molekulargewicht von etwa 1000 bis 100.000 aktiviert wird durch Spaltung eines Aldohexopyranoserings oder Koppelung mit einer bifunktionellen Verbindung aktiviert und das aktivierte Polysaccharid dann mit dem Antigen direkt oder über einen 1 bis 3 C-Atome enthaltenden Spacer gekuppelt wird.

Aus US-A-2 609 368 ist ein Verfahren zur Herstellung von Cellulosaten durch Umsetzung des Cellulosematerials mit einer wasserfreien alkoholischen Alkalimetallalkoholatlösung bei einer Temperatur von 80 bis 115°C bekannt. Die gebildeten 2-Monoalkaliglucopyranosepolymere können mit organischen Reaktanten wie insbesondere Halogeniden, zu in Position 2 substituierten Glucopyranosepolymerethern umgesetzt werden.

Die bisher bekannten Verfahren zur Bindung von Haptenen an eine Matrix erwiesen sich alle als nicht optimal. Die Bindung der Haptene an die Matrix war nicht ausreichend stark, so daß der Träger bei Anwendung der für die einzelnen Verfahren notwendigen Bedingungen ausblutete, d. h. das Hapten abgab.

Ein anderes Problem bei der Bindung des Haptens an einen Träger besteht darin, daß das Hapten so gebunden werden muß, daß seine aktive Stelle nicht blockiert wird, so daß seine Aktivität auch nicht teilweise verloren geht.

Es war nun Aufgabe der vorliegenden Erfindung, ein Verfahren zur Verfügung zu stellen, mit dem Haptene an einen Träger fixiert werden können, ohne daß sie ihre Aktivität verlieren und ohne daß sie bei Anwendung der bei Immunosssays oder Affinitätschromatographie erforderlichen Bedingungen von dem Träger abgelöst werden.

Diese Aufgabe wird gelöst durch ein Verfahren zur Herstellung einer Polysaccharidmatrix, an die Haptene kovalent gebunden sind, welches dadurch gekennzeichnet ist, daß man ein in Wasser praktisch unlösliches Polysaccharid enthaltendes Material alkalisiert, auf einen Wassergehalt von weniger als 5 % trocknet und dann in wasserfreiem Medium mit einer mindestens eine aktivierte funktionelle Gruppe enthaltendem im humanen Immunsystem als Hapten wirkenden Substanz umsetzt.

Überraschenderweise wurde festgestellt, daß bei Anwendung der erfindungsgemäßen Verfahrensschritte das Hapten einerseits so stark an die Matrix gebunden wird, daß es auch bei Anwendung drastischer Bedingungen nicht abgelöst wird. Andererseits erfolgt die Bindung des Haptens so, daß es seine Aktivität nicht verliert.

Das erfindungsgemäße Verfahren dient zur Herstellung einer haptenisierten Polysaccharidmatrix. Dazu wird polysaccharidhaltiges Material verwendet. Als Polysaccharid geeignet sind die leicht zugänglichen, in Wasser praktisch unlöslichen Polysaccharide wie Agarose, Dextran oder Cellulose. Diese Polysaccharide können mit anderen inerten Bestandteilen vermischt sein. Bevorzugt wird als Polysaccharid Cellulose eingesetzt. Geeignete cellulosehaltige Materialien sind reine Cellulose, Sulfitzellstoff, Zellwolle und/oder Celluloseacetat sowie Mischungen dieser Komponenten. Das Material kann weiterhin noch mit anderen Fasern, die in solchen Materialien üblicherweise verwendet werden, vermischt werden. Geeignet sind beispielsweise Polyester, Nylon oder Polyacrylnitril.

Die Eigenschaften des polysaccharidhaltigen Materials können je nach Verwendungszweck variiert werden. So können Hydrophobizität, Oberflächeneigenschaften etc. durch Verwendung geeigneter Polysaccharid-Derivate eingestellt werden. Unterschiedliche Hydrophobizität kann beispielsweise durch Verwendung von Celluloseacetat mit unterschiedlichem Acetylierungsgrad zur Herstellung der Matrix erreicht werden.

Bevorzugt werden Materialien verwendet, die einen hohen Anteil an Polysaccharid haben.

Das polysaccharidhaltige Material wird zuerst alkalisiert. Dabei wird durch Einwirken basischer Substanzen zumindest ein Teil der OH-Gruppen des Polysaccharids in die aktivere Salzform umgewandelt. Geeignet zur Alkalisierung sind z.B. wäßrige Laugen oder in organischem Lösungsmittel gelöste Alkoholate. Bevorzugt werden zur Alkalisierung Alkalihydroxide oder Alkalialkoholate gelöst in dem korrespondierenden Alkohol eingesetzt. Besonders bevorzugt wird wäßrige Natronlauge oder Natriummethanolat, gelöst in Methanol, verwendet.

Die Alkalikonzentration ist an sich nicht kritisch, die optimale Konzentration richtet sich jedoch nach der eingesetzten Menge an Hapten. Wird bezogen auf Polysaccharidmaterial viel Hapten eingesetzt, so sollte auch die Alkalimenge entsprechend größer sein und umgekehrt sollte bei Einsatz geringer Haptenmengen auch die Alkalimenge reduziert werden. Die jeweils optimalen Einsatzmengen lassen sich durch wenige Vorversuche feststellen. Bevorzugt wird die Lauge in einer Konzentration von 0,005 bis 5n, besonders bevorzugt 0,01 bis 1n, insbesondere 0,05 bis 0,2n verwendet.

Im Anschluß an die Alkalisierung wird das polysaccharidhaltige Material sorgfältig getrocknet bis auf einen Restwassergehalt von höchstens 5%. Bevorzugt wird das Material so weit getrocknet, daß der Wassergehalt weniger als 2 Gew.-% beträgt. Die Trocknung erfolgt in an sich bekannter Weise, wobei die Trocknungsbedingungen so sein müssen, daß das polysaccharidhaltige Material nicht geschädigt wird.

Wurde zur Alkalisierung ein Alkoholat, gelöst in Alkohol verwendet, so ist die Trocknung nicht erforderlich.

Das alkalisierte, getrocknete, polysaccharidhaltige Material wird dann in wasserfreiem Medium mit dem Hapten umgesetzt. Die Umsetzung erfolgt daher vorzugsweise in wasserfreiem organischem Lösungsmittel wie Aceton, DMF oder Dioxan.

Anschließend wird mit organischen Lösungsmitteln gewaschen und mit wässriger Pufferlösung mit pH 6 bis 8 und gegebenenfalls mit Wasser nachgewaschen.

Geeignet zur Umsetzung mit dem polysaccharidhaltigen Material sind alle im menschlichen Immunsystem als Haptene wirksamen Substanzen, die eine funktionelle Gruppe besitzen oder bei denen leicht eine funktionelle Gruppe eingeführt werden kann. Bevorzugt wird als Hapten $T_3$, $T_4$, Digoxin, Diphenylhydantoin, Folat und/oder Biotin verwendet.

Das Hapten wird an das Polysaccharid über eine aktivierte funktionelle Gruppe gebunden. Falls das verwendete Hapten bereits eine funktionelle Gruppe, die für die Bindung geeignet ist, besitzt, so wird diese aktiviert und anschließend erfolgt die Umsetzung. Besitzt das Hapten keine geeignete funktionelle Gruppe, so wird nach an sich bekannten Methoden eine funktionelle Gruppe in das Hapten eingeführt. Geeignete funktionelle Gruppen für die Bindung mit dem Polysaccharid sind insbesondere Carboxyl-, Hydroxyl- und Aminogruppen, geeignet zur Einführung einer funktionellen Gruppe sind insbesondere in $\omega$-Position reaktionsfähige Carbonsäuren, die z.B. über Oxim- und Amidbildung oder Alkylierung an das Hapten gebunden werden.

Die Aktivierung der funktionellen Gruppe erfolgt ebenfalls nach an sich bekannten Methoden. Dabei wird die Carboxylgruppe so derivatisiert, daß sie leicht mit den $O^-$-Gruppen des Polysaccharid-haltigen Materials reagieren kann. So sind Möglichkeiten zur Aktivierung beschrieben in K. Lübke, E. Schröder, G. Kloss, Chemie und Biochemie der Aminosäuren, Peptide und Proteine I, G. Thieme Verlag, Stuttgart, 1975, S. 136 bis 137. Bei der Durchführung des erfindungsgemäßen Verfahrens sind bevorzugte Varianten für die Aktivierung der Carboxylgruppe, die Imidazolid- und die Säurechloridmethode, sowie die Methode der aktivierten N-Hydroxyester unter Verwendung von N-Hydroxyimiden und die Methode der gemischen Carbonsäureanhydride. Besonders bevorzugt wird erfindungsgemäß ein aktivierter N-Hydroxyester verwendet, wobei insbesondere die Hydroxysuccinimidester zum Einsatz kommen.

Enthält das Hapten als funktionelle Gruppe eine Aminogruppe, so erfolgt die Aktivierung bevorzugt mit Bromessigsäurechlorid. Zur Aktivierung einer Hydroxylgruppe wird bevorzugt eine gegebenenfalls z.B. als Anhydrid aktivierte Dicarbonsäure verwendet.

Das aktivierte Hapten wird in wasserfreiem Medium mit dem Polysaccharid-haltigen Material umgesetzt. Die jeweils optimalen Mengen der eingesetzten Komponenten richten sich nach Art und Verwendungszweck des Materials. Als geeignet hat sich ein Verhältnis von Polysaccharidhaltigem Material zu Hapten im Bereich von $10^4$ bis 0,1:1 erwiesen. Bevorzugt beträgt das Verhältnis von Polysaccharid-haltigem Material zu Hapten $10^3$ bis 10:1.

Die bevorzugten Konzentrationen liegen im Bereich von $10^{-4}$ bis 10 g/l, besonders bevorzugt $10^{-3}$ bis $10^{-1}$ g/l.

3

Das erfindungsgemäß hergestellte Polysaccharid-haltige Material, an das Haptene kovalent gebunden sind, wird in an sich bekannter Weise je nach Verwendungszweck verarbeitet. Für den Einsatz in der Affinitätschromatographie liegt das Material bevorzugt in Form von Kugeln oder Pulver vor. Für den Einsatz in Immunoassays wird das Polysaccharid-haltige Material bevorzugt in Form von Filmen, Vliesen oder Papier verwendet. Es ist nun einerseits möglich, das Polysaccharid-haltige Material zuerst mit dem aktivierten Hapten umzusetzen und anschließend das haptenisierte Material in die gewünschte Form zu bringen. Andererseits ist es ebenfalls möglich, zuerst das Polysaccharid-haltige Material in die gewünschte Form zu bringen und anschließend mit dem aktivierten Hapten umzusetzen. So können beispielsweise zuerst Cellulosefasern haptenisiert werden und anschließend dann die Fasern zu einem Vlies verarbeitet werden. Andererseits können zuerst aus dem Polysaccharidhaltigen Material Kugeln geformt werden, die dann anschließend haptenisiert werden.

Die Umsetzung des alkalisierten und getrockneten Polysaccharid-haltigen Materials mit dem aktivierten Hapten erfolgt bevorzugt in einer Vorrichtung, die gekennzeichnet ist durch einen Wickelkern in einem Behälter für Reaktionslösung, welcher ein in seiner Wand mit einer Vielzahl von Öffnungen versehenes, an einem Ende verschlossenes Rohr für die Aufnahme eines Wickels des zu behandelnden Materials und zwei axialen Wangenscheiben zur Abdichtung der axialen Stirnseiten des Wickels aufweist und durch einen Umwälzpumpenkreis, der die Reaktionslösung aus dem Behälter dem anderen Ende des Rohrs zuführt.

Bevorzugt sind dabei die Wangenscheiben gegen die axialen Stirnseiten des Wickels spannbar.

In einer bevorzugten Ausführungsform wird nun in den Behälter die das aktivierte Hapten enthaltende Reaktionslösung eingefüllt. Das alkalisierte und getrocknete Polysaccharid-haltige Material, das bahnenförmig vorliegt, wird auf den Wickelkern aufgewickelt und mit einer Gaze fixiert. Der Papierrand wird durch die Wangenscheiben abgedichtet. Die Reaktionslösung wird dann über den Umwälzpumpenkreis von dem Behälter dem anderen Ende des Rohrs zugeführt. Dabei durchströmt die Reaktionslösung das aufgewickelte Material nach allen Richtungen radial von innen nach außen. Vorteil dieser Vorrichtung ist es, daß nur wenig organisches Lösungsmittel verwendet werden muß. Weiterhin ist die Apparatur sehr leicht zu handhaben und zu reinigen.

Mit dem erfindungsgemäßen Verfahren kann unter Verwendung leicht verfügbarer Einsatzstoffe eine vielseitig verwendbare Matrix im Produktionsmaßstab homogen und reproduzierbar hergestellt werden, die sowohl für die Chromatographie als auch z.B. als spezifische Matrix oder als Universalmatrix in Immunoassays eingesetzt werden kann. Dabei dient die erfindungsgemäß hergestellte haptenisierte Matrix als feste Phase. Diese erfindungsgemäße haptenisierte Matrix ist in zwei verschiedenen Varianten für Bestimmungsverfahren nach dem Prinzip des Immunoassays einsetzbar.

Einerseits kann an die Matrix ein Hapten gebunden werden, das ein Konjugat aus einer zu bestimmenden Substanz und einer damit spezifisch bindefähigen markierten Substanz quantitativ und spezifisch binden kann. So kann beispielsweise $T_3$ oder $T_4$ an der Matrix immobilisiert werden und damit dann ein Konjugat, das einen gegen $T_3$ bzw. $T_4$ gerichteten Antikörper enthält, gebunden werden.

Eine andere Verfahrensvariante besteht darin, daß an das Hapten wiederum eine spezifisch bindefähige Substanz gebunden wird, die wiederum mit derivatisierten Konjugaten aus zu bestimmender Substanz und damit bindefähiger markierter Substanz reagieren kann. So kann beispielsweise an der Matrix Biotin immobilisiert werden, und dann ein mit Avidin konjugierter Antikörper gebunden werden. An die immobilisierten Biotinmoleküle kann spezifisch Biotin bindendes Streptavidin oder Avidin gebunden werden. Eine derartig präparierte Matrix kann dann als Universalmatrix eingesetzt werden und mit jedem biotinylierten Antikörper oder einer sonst spezifisch bindefähigen biotinylierten Substanz reagieren.

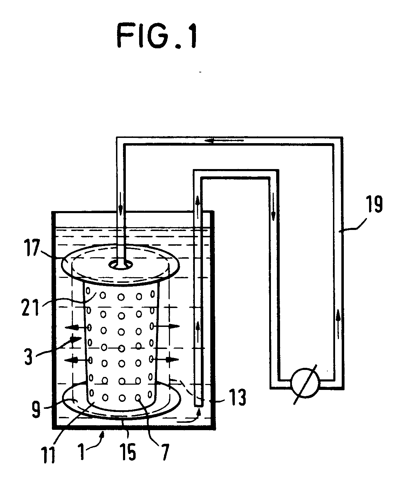

Die Erfindung wird durch die Figur 1 und durch die Beispiele erläutert.

Fig. 1    zeigt eine Vorrichtung zur Durchführung des erfindungsgemäßen Verfahrens.

Fig. 1 zeigt einen Behälter für Reaktionslösung 1, in dem sich ein Wickelkern 3 befindet. Der Wickelkern weist ein an einem Ende 9 verschlossenes Rohr 11 für die Aufnahme eines Wickels 13 des zu behandelnden Materials auf, wobei das Rohr 11 eine Vielzahl von Öffnungen 7 aufweist. An den axialen Stirnseiten des Wickels 13 sind zwei axiale Wangenscheiben 15, 17 zur Abdichtung angeordnet, die gegen die axialen Stirnseiten des Wickels 13 spannbar sind. Die Vorrichtung weist weiterhin einen Umwälzpumpenkreis 19 auf, der die Reaktionslösung aus dem Behälter 1 dem anderen Ende (21) des Rohrs 11 zuführt.

## Beispiel 1

25 m eines cellulosehaltigen Papiers werden auf einer Papierimprägnieranlage durch Wannentränkung mit 0,1n NaOH behandelt. Nach Aufnahme der Alkalilösung wird das alkalisierte Papier auf der Papierimprägnieranlage durch Luftumwälzung bei 50 bis 80 °C getrocknet.

Als Hapten wird Trijodthyronin, $T_3$, verwendet. Die Aminogruppe des $T_3$ wird durch einen tert.-Butyloxycarbonylrest (BOC) geschützt. Die COOH-Gruppe wird aktiviert durch Umwandlung in den Hydroxy-Succinimidester. Es wird eine Lösung von 25 mg Boc-$T_3$-N-Hydroxy-Succinimidester in 4 l Aceton hergestellt.

Die Veresterung des derivatisierten $T_3$ mit dem alkalisierten cellulosehaltigen Papier wird in einem Reaktor durchgeführt, wie er in Fig. 1 dargestellt ist. Dabei wird das alkalisierte Papier auf einer Papierrollenschneidemaschine satt und bündig auf den Wickelkern des Reaktors gewickelt und mit einer Nylongaze fixiert. Der Papierrand wird durch sattes Anschrauben der Abdichtwangen abgedichtet. Der mit dem alkalisierten Papier umwickelte Kern wird in das Reaktionsgefäß eingebracht, das die Reaktionslösung enthält. Die Reaktionslösung wird dann mit einer Durchflußgeschwindigkeit von 2,5 l/min mit der Pumpe über die Schlauchleitungen umgepumpt und durchströmt das Papier nach allen Richtungen radial von innen nach außen.

Nach einer Reaktionszeit von 1,5 Stunden bei Raumtemperatur wird die Reaktionslösung entfernt und es werden 4 l Aceton bei Raumtemperatur 5 Minuten zum Waschen umgepumpt. Anschließend wird 10 Minuten bei Raumtemperatur mit einer Pufferlösung, die 0,1 Mol/l Phosphatpuffer und 1 %o Tween 20 enthält und einen pH von 7 hat, gewaschen. Zwei weitere Waschungen mit diesem Puffer bei jeweils 50 °C über 10 Minuten schließen sich an. Anschließend wird 5 Minuten bei Raumtemperatur mit Wasser gewaschen.

Das so erhaltene Papier wird durch Umpumpen von je 4 l Aceton über 5 Minuten im Reaktor entwässert. Dann wird eine Endtrocknung auf der Papierimprägnieranlage bei 50 °C im Umluftverfahren bis zu einer Restfeuchte von 1,5 bis 2% durchgeführt.

**Beispiel 2**

An cellulosehaltiges Papier wird $T_4$ als Hapten gebunden. Das Verfahren erfolgt wie im Beispiel 1 beschrieben, jedoch wird statt BOC-$T_3$-Hydroxy-Succinimidester als Reaktionslösung eine Lösung eingesetzt, die 100 mg BOC-$T_4$-N-Hydroxy-Succinimidester enthält.

**Beispiel 3**

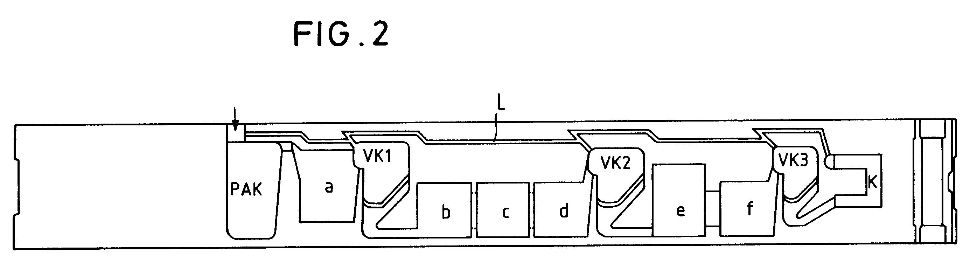

Bestimmung der Bindekapazität einer $T_3$-Matrix unter Verwendung eines Einsatzelements gemäß Fig. 2 in einem Gerät gem. EP 0167171 und 0167175.

Bestimmung des Einsatzelements:

a: Vlies aus 40% Linters 941, 50% Polyamid, 10% Kuralon
30 mmol/l $Na_3 PO_4$ x $12H_2O$
0,5 o/oo Genapol PF20
b: Leerkammer (kein Vlies)
c: Vlies aus 20% Linters 941, 30% Zellwolle, 40% Polyamid 8,2/4, 10% Kuralon,
getränkt mit einer Lösung von:
38 mmol/l $Na_2H PO_4$ x $2 H_2O$
36 mmol/l $Na H_2 PO_4$ x $H_2O$
3,5 mmol/l $MgK_2$ EDTA x $2 H_2O$
0,7% Rinderserumalbumin
0,7% Crotein C
0,035% Anilinonaphtylsulfonsäure (ANS)
0,01% Genapol PF 20
d: Vlies aus 80% Polyester, 20% Zellwolle
getränkt mit einer Lösung von:
280 U/l polyklonale Antikörper gegen $T_3$, gekoppelt an $\beta$-Galactosidase.
100 mmol/l 4-(2-Hydroxyethyl)-1-piperazinethanolsulfonsäure (HEPES) pH 7,25
5 mmol/l Mg-L-Aspartat
1% Crotein C
zur Lagerung wird das getränkte Vlies lyophilisiert.
e: Vlies analog Beispiel 1 (Matrix)
f: Vlies aus 20% Linters, 30 % Zellwolle, 40 % Polyamid, 10 % Kuralon
getränkt mit einer Lösung von:

19 mmol/l Chlorphenolrot-galactosid
100 mmol/l HEPES pH 7,25
10 mmol/l Borsäure

Flüssigkeitspipettierungen

Als Probe werden in die Probenauftragskammer (PK) 56 $\mu$l einer 0,9 Gew.-% NaCl-Lösung pipettiert.

Reaktionsdurchführung

Wenn die Probe in die Probenauftragskammer pipettiert ist, beginnt das Zentrifugationsprogramm. Durch das Zusammenwirken von Schwerkraft, Kapillarkräften und Zentrifugalkraft wird die Probenlösung durch die verschiedenen Reagenzträger hindurch bis in die Küvette transportiert. Um die zeitliche Abfolge der Reaktionsschritte zu steuern, wird das in der Tabelle 1 dargestellte Zentrifugenprogramm verwendet. In dieser Tabelle sind Schrittnummern, Dauer des jeweiligen Zentrifugenschrittes in Sekunden, Drehzahl in rpm und eine Beschreibung der Funktion dieser Schritte angegeben.

Zur Messung (Schritt 26) wird die Lösung in die Küvette transportiert und die Reaktion absorptions-photometrisch bei 578 nm verfolgt. Die Konjugatmoleküle, welche nicht von der Matrix (Vlies e) gebunden werden, konnten die Matrix passieren und es befindet sich jetzt eine entsprechende Menge Enzym in der Küvette. Die gemessene Farbzunahme pro Zeiteinheit (Extinktion/min, mE/min) ist somit ein Maß für die Bindekapazität der Matrix. Je geringer die gemessene Farbzunahme pro Minute, desto größer ist die Bindekapazität der Matrix.

Tabelle 1                    Zentrifugationsprogramm

| STEP | SEC | RPM | |
|------|-----|-----|---|
| 1 | 60 | 100 | Temperierung Dispo mit Probe in PAK |
| 2 | 5 | 200 | Probe auf Puffervlies 1 in Feld a |
| 3 | 20 | 400 | Elution des Puffervlieses |
| 4 | 15 | 3500 | |
| 5 | 45 | 600 | Mischen und Inkubation in VK 1 |
| 6 | 40 | 0 | Auslauf VK 1 |
| 7 | 5 | 100 | Probe auf Puffervlies 2 in Feld c |
| 8 | 25 | 250 | Probe auf Konjugatvlies in Feld d |
| 9 | 15 | 3500 | Elution von Puffervlies 1 und Konjugatvlies |
| 10 | 140 | 600 | Homogene Reaktion in VK 2 |
| 11 | 130 | 600 | |
| 12 | 60 | 0 | Auslauf VK 2 |
| 13 | 5 | 100 | Probe auf Matrixvlies in Feld e |
| 14 | 5 | 300 | Matrix Reaktion |
| 15 | 120 | 100 | |
| 16 | 120 | 100 | |
| 17 | 2 | 400 | Ausschleudern der Matrix |
| 18 | 2 | 600 | Trennschritt gebundenes freies Konjugat |
| 19 | 2 | 800 | Probe auf Substratvlies in Feld f |
| 20 | 2 | 1200 | dadurch Start der Indikatorreaktion |
| 21 | 0 | 100 | Start TIMER |
| 22 | 10 | 3500 | Ausschleudern Matrix- und Substratvlies |
| 23 | 15 | 0 | Auslauf VK 3 |
| 24 | 2 | 100 | |
| 25 | 2 | 300 | Füllen der Küvette |
| 26 | 60 | 720 | Messung  (Wellenlänge $\lambda$ = 578 nm) |
| 27 | 3600 | 3600 | Ende |

Fig. 2 zeigt ein Dispo, wie es zur Durchführung von Beispiel 3 verwendet wird. Dabei bedeuten:

PAK: Probenauftragskammer

VK1, VK2, VK3: Ventilkammern

K: Meßküvette

L: Lüftungskanal

Die einzelnen Vliese und Kammern sind schematisch dargestellt. Dabei bedeuten Stege, die den Kontakt zwischen den einzelnen Kammern und Vliesen herstellen, jeweils Verbindungen durch die die zu untersuchende Flüssigkeit weitertransprotiert werden kann.

**Beispiel 4**

In einem alternativen Verfahren wurde die Bindekapazität einer $T_3$-Matrix bestimmt. Dazu wurden die folgenden Reagenzien verwendet:

a) Puffer:

38 mmol/l $Na_2HPO_4 \cdot 2 H_2O$

36 mmol/l $NaH_2PO_4 \cdot H_2O$

3,5 mmol/l $MgK_2$ EDTA $\cdot 2 H_2O$

0,7 % Rinderserumalbumin

0,7 % Crotein C

0,035 % Anilinonaphthylsulfonsäure (ANS)

0,01 % Genapol PF 20

b) Chlorphenolrotgalactosidlösung (19 mmol/l) in Puffer (100 mmol/l Hepes, pH 7,25; 10 mmol/l Borsäure)

c) Anti-$T_3$-Antikörper-$\beta$-Gal-Konjugat

Lösung eines Konjugats aus polyklonalem Antikörper gegen $T_3$ mit $\beta$-Galactosidase. ($\beta$-Gal) ($\beta$-Galactosidase-Aktivität: 280 U/l) in Puffer a)

Zur Durchführung des Tests wurde die Probe (0,9 Gew.% NaCl-Lösung) im Volumenverhältnis 1 : 10 mit Anti-$T_3$-Antikörper-$\beta$-Gal-Konjugat-Lösung verdünnt. 50 $\mu$l dieses Gemisches wurden von 10 mg des $T_3$ tragenden Papiers (Herstellung nach Beispiel 1) aufgesaugt und 10 Minuten bei 37°C inkubiert. Zur Entfernung von ungebundenem Konjugat wurde das Papier dreimal mit je 100 $\mu$l Puffer gewaschen, wobei nach jedem Waschvorgang die Waschlösung durch Zentrifugation entfernt wurde. Anschließend wurde das Papier mit 50 $\mu$l Chlorphenolrotgalactosidlösung getränkt. Nach 5 Minuten Inkubation bei 37°C wurde die gebildete Farbstofflösung durch Zentrifugation vom Papier abgetrennt und in einer Mikroküvette mit einem Füllvolumen von 27 $\mu$l und einer Schichtdicke von 0,6 cm bei einer Wellenlänge von 578 nm gemessen. Die gemessene Extinktion ist der Bindungskapazität der Probe umgekehrt proportional.

**Beispiel 5**

An cellulosehaltiges Papier wurde Biotin als Hapten gebunden. Das Verfahren wurde durchgeführt, wie im Beispiel 1 beschrieben. Statt BOC-$T_3$-Hydroxy-Succinimidester wurde als Reaktionslösung eine Lösung verwendet, die 200 mg Biotin-N-Hydroxy-Succinimidester enthielt.

**Beispiel 6**

Es wird ein mit $T_3$ derivatisiertes cellulosehaltiges Papier hergestellt. Das Verfahren erfolgt wie im Beispiel 1 beschrieben. Jedoch wird ein anderes $T_3$-Derivat eingesetzt. Dazu wird $T_3$ mit $CH_2Br$-COCl derivatisiert zu $BrCH_2CO$-$T_3$. 5 mg $BrCH_2CO$-$T_3$ in 125 ml Dioxan werden zu 2 g alkalisiertem Papier gegeben und über Nacht bei Raumtempratur geschüttelt. Anschließend wird zweimal mit je 50 ml Dioxan, Aceton und Wasser gewaschen. Das so behandelte Papier wird über 12 Stunden mit 2,5 l 20 mM Phosphatpuffer mit pH 8 in einer Säule unter langsamem Durchspülen behandelt, anschließend mit je 50 ml $H_2O$ und Aceton behandelt und im Vakuum getrocknet.

**Beispiel 7**

Es wurde untersucht, inwieweit die Bindekapazität der Cellulose für ein Hapten durch die Trocknung beeinflußt wird. Dazu wurde alkalisierte Cellulose, an der $T_3$ immobilisiert war, unter unterschiedlichen Bedingungen getrocknet und anschließend mit BOC-$T_3$-N-Hydroxy-Succinimidester, wie im Beispiel 1 beschrieben, umgesetzt. Die Ergebnisse sind der folgenden Tabelle zu entnehmen. Hierbei ist die Bindekapazität umgekehrt proportional zum Extinktionswert (Bestimmung nach Beispiel 3):

| Trocknung | Extinktionswert für $T_3$ (mE/min) |
|---|---|
| feuchtes Papier | 550 |
| mit Aceton getrocknet und auf ca. 13 % Wassergehalt getrocknet | 450 |
| 2 Stunden bei 25 °C im Umlufttrockenschrank auf ca. 8 % Wassergehalt getrocknet | 212 |
| über Nacht im Exsikator auf 1% Wassergehalt getrocknet | 75 |

**Beispiel 8**

Es wurde der Einfluß des Wassergehalts des Reaktionsmediums auf die Bindekapazität getestet. Dazu wurden 0,33 mg BOC-$T_3$-N-Hydroxy-Succinimidester, pro g Papier, gelöst in 2 mg/l Aceton, wie im Beispiel 3 beschrieben, an das Papier gekuppelt. Die Bestimmung der Bindekapazität erfolgte wie im Beispiel 4 beschrieben. Zu einem Papier, welches nach Waschen und Trocknen gemäß Beispiel 1 zusätzlich im Vakuumtrockenschrank bei 50 °C/10 bis 2 Torr 8 Stunden getrocknet wurde, wurden zur Prüfung des Einflusses des Wassergehaltes die in der Tabelle angegebenen Wassermengen zugegeben. Die Prozentangabe bedeutet Gew.-% vom Lösungsmittel, das das Reaktionsmedium darstellt. Die Ergebnisse sind der folgenden Tabelle zu entnehmen.

| % $H_2O$ | Bindekapazität (mE/min) |
|---|---|
| 0,2 | 47 |
| 0,5 | 48 |
| 1,0 | 65 |
| 10 | 1298 |

**Beispiel 9**

Es wurde wie im Beispiel 1 beschrieben, ein Papier hergestellt, an das Diphenylhydantoin gekuppelt war. Dazu wurden 1 mg Diphenylhydantoin-Valeriansäure-N-Hydroxy-Succinimidester pro g Papier verwendet. Es wurde eine Reaktionslösung hergestellt, die 6 mg Diphenylhydantoin-Valeriansäure-Succinimidester pro 1 Aceton enthielt.

**Patentansprüche**

1. Verfahren zur Herstellung einer Polysaccharidmatrix, an die Haptene kovalent gebunden sind, **dadurch gekennzeichnet,** daß man ein in Wasser praktisch unlösliches Polysaccharid enthaltendes Material alkalisiert, auf einen Wassergehalt von weniger als 5 % trocknet und dann in wasserfreiem Medium mit einer mindestens eine aktivierte funktionelle Gruppe enthaltendem im humanen Immunsystem als Hapten wirkenden Substanz umsetzt.

2. Verfahren nach Anspruch 1, **dadurch gekennzeichnet,** daß man als Polysaccharidmaterial cellulosehaltiges Material verwendet.

3. Verfahren nach Anspruch 2, **dadurch gekennzeichnet,** daß man als cellulosehaltiges Material Cellulose, Sulfitzellstoff, Zellwolle und/oder Celluloseacetat und/oder Mischungen davon verwendet.

4. Verfahren nach Anspruch 1 bis 3, **dadurch gekennzeichnet,** daß man das Polysaccharid-haltige Material zur Alkalisierung mit wäßriger 0,005 bis 5n Alkali-hydroxid behandelt.

5. Verfahren nach einem der Ansprüche 1 bis 3, **dadurch gekennzeichnet,** daß man die Alkalisierung mit einem Alkali-Alkoholat durchführt.

**6.** Verfahren nach einem der vorhergehenden Ansprüche, **dadurch gekennzeichnet,** daß man das Polysaccharid-haltige Material nach der Alkalisierung bis auf einen Wassergehalt von weniger als 2% trocknet.

**7.** Verfahren nach einem der vorhergehenden Asprüche, **dadurch gekennzeichnet,** daß man ein Hapten verwendet, das als funktionelle Gruppe eine Carboxyl-, Amino- oder Hydroxylgruppe trägt.

**8.** Verfahren nach Anspruch 7, **dadurch gekennzeichnet,** daß die Aktivierung der Carboxylgruppe mit einem Säurechlorid, einem Hydroxyimid, einem Carbodiimid, einem Anhydrid oder einem Imidazolid erfolgt.

**9.** Verfahren nach einem der vorhergehenden Ansprüche, **dadurch gekennzeichnet,** daß man Polysaccharid-haltiges Material und Hapten in einem Verhältnis von $10^4$ bis 0,1:1 einsetzt.

**10.** Verfahren nach einem der vorhergehenden Ansprüche, **dadurch gekennzeichnet,** daß man als Hapten gegebenenfalls derivatisiertes $T_3$, $T_4$, Digoxin, Diphenylhydantoin, Folat oder Biotin verwendet.

**11.** Vorrichtung zur Durchführung des Verfahrens nach einem der Ansprüche 1 bis 10, **gekennzeichnet durch** einen Wickelkern (3) in einem Behälter für Reaktionslösung (1), welcher ein in seiner Wand (5) mit einer Vielzahl von Öffnungen (7) versehenes, an einem Ende (9) verschlossenes Rohr (11) für die Aufnahme eines Wickels (13) des zu behandelnden Materials und zwei axialen Wangenscheiben (15, 17) zur Abdichtung der axialen Stirnseite des Wickels (13) aufweist und durch einen Umwälzpumpenkreis (19), der die Reaktionslösung aus dem Behälter (1) dem anderen Ende (21) des Rohrs (11) zuführt.

**12.** Vorrichtung nach Anspruch 11, **dadurch gekennzeichnet,** daß die Wangenscheiben (15, 17) gegen die axialen Stirnseiten des Wickels (13) spannbar sind.

**Claims**

**1.** Process for the production of a polysaccharide matrix to which haptens are covalently bound, characterised in that one alkalises polysaccharide-containing material which is practically insoluble in water, dries to a water content of less than 5% and then reacts in anhydrous medium with a substance acting as hapten in the human immune system containing at least one activated functional group.

**2.** Process according to claim 1, characterised in that one uses cellulose-containing material as polysaccharide material.

**3.** Process according to claim 2, characterised in that, as cellulose-containing material, one uses cellulose, sulphite wood pulp, viscose staple and/or cellulose acetate and/or mixtures thereof.

**4.** Process according to claims 1 to 3, characterised in that, for the alkalisation, one treats the polysaccharide-containing material with aqueous 0.005 to 5N alkali metal hydroxide.

**5.** Process according to any of claims 1 to 3, characterised in that one carries out the alkalisation with an alkali metal alcoholate.

**6.** Process according to one of the preceding claims, characterised in that, after the alkalisation, one dries the polysaccharide-containing material to a water content of less than 2%.

**7.** Process according to one of the preceding claims, characterised in that one uses a hapten which, as functional group, contains a carboxyl, amino or hydroxyl group.

**8.** Process according to claim 7, characterised in that the activation of the carboxyl group takes place with an acid chloride, a hydroxyimide, a carbodiimide, an anhydride or an imidazolide.

9. Process according to one of the preceding claims, characterised in that one uses the polysaccharide-containing materials and hapten in a ratio of $10^4$ to 0.1:1.

10. Process according to one of the preceding claims, characterised in that, as hapten, one uses optionally derivatised $T_3$, $T_4$, digoxin, diphenylhydantoin, folate or biotin.

11. Device for the carrying out of the process according to one of claims 1 to 10, characterised by a winding core (3) in a container for reaction solution (1) which has a tube (11), provided in its wall (5) with a plurality of openings (7), closed on one end (9), for the reception of a winding (13) of the material to be treated and two axial end walls (15, 17) for the sealing off of the axial front sides of the winding (13) and by a circulating pump cycle (19) which feeds the reaction solution from the container (1) to the other end (21) of the tube (11).

12. Device according to claim 11, characterised in that the end walls (15, 17) can be stressed against the axial front sides of the winding (13).

**Revendications**

1. Procédé de préparation d'une matrice de polysaccharide à laquelle des haptènes sont fixés de manière covalente, caractérisé en ce que l'on alcalinise un matériau contenant un polysaccharide pratiquement insoluble dans l'eau, on le sèche jusqu'à une teneur en eau inférieure à 5% puis on le fait réagir en milieu anhydre avec une substance qui contient au moins un groupe fonctionnel activé et qui agit comme un haptène dans le système immunitaire humain.

2. Procédé selon la revendication 1, caractérisé en ce que l'on utilise un matériau contenant de la cellulose comme matériau contenant un polysaccharide.

3. Procédé selon la revendication 2, caractérisé en ce que l'on utilise comme matériau contenant de la cellulose la cellulose, la pâte au sulfite, la laine de cellulose et/ou l'acétate de cellulose et/ou des mélanges de ces-ci.

4. Procédé selon les revendications 1 à 3, caractérisé en ce que l'on traite le matériau contenant un polysaccharide avec un hydroxyde alcalin aqueux 0,005 à 5 N pour l'alcalinisation.

5. Procédé selon l'une des revendications 1 à 3, caractérisé en ce que l'on accomplit l'alcalinisation avec un alcoolate alcalin.

6. Procédé selon l'une des revendications précédentes, caractérisé en ce que l'on sèche le matériau contenant un polysaccharide jusqu'à une teneur en eau inférieure à 2% après l'alcalinisation.

7. Procédé selon l'une des revendications précédentes, caractérisé en ce que l'on utilise un haptène qui porte comme groupe fonctionnel un groupe carboxyle, aminé ou hydroxyle.

8. Procédé selon la revendication 7, caractérisé en ce que l'activation du groupe carboxyle se fait avec un chlorure d'acide, un hydroxyimide, un carbodiimide, un anhydride ou un imidazolide.

9. Procédé selon l'une des revendications précédentes, caractérisé en ce que l'on utilise le matériau contenant un polysaccharide et l'haptène dans un rapport de $10^4$ à 0,1:1.

10. Procédé selon l'une des revendications précédentes, caractérisé en ce que l'on utilise comme haptène $T_3$, $T_4$, la digoxine, la diphénylhydantoine, un folate ou la biotine éventuellement dérivatisé.

11. Dispositif pour la mise en oeuvre du procédé selon l'une des revendications 1 à 10, caractérisé par un noyau de rouleau (3) dans un récipient pour solution réactionnelle (1), qui présente un tube (11) muni d'une multiplicité d'ouvertures (7) dans sa paroi (5) et fermé à une extrémité (9) pour recevoir un rouleau (13) du matériau à traiter et deux disques latéraux (15, 17) axiaux pour étanchéifier le côté frontal axial du rouleau (13) et par un circuit à pompe de circulation (19) qui envoie la solution réactionnelle du récipient (1) à l'autre extrémité (21) du tube (11).

**12.** Dispositif selon la revendication 11, caractérisé en ce que les disques latéraux (15, 17) peuvent être serrés contre les côtés frontaux axiaux du rouleau (13).

# FIG.1

FIG. 2